# EUROPEAN PATENT SPECIFICATION

(11) **EP 3 379 251 B1**
(45) Date of publication and mention of the grant of the patent: **25.12.2019**
(21) Application number: 18163229.0
(22) Date of filing: 22.03.2018
(51) Int. Cl.: G01N 33/558, G01N 33/68

(54) **MEDICAL DEVICE, IMMUNOASSAY METHOD AND TEST FOR DETECTING PROLIFERATIVE DIABETIC RETINOPATHY**
MEDIZINISCHE VORRICHTUNG, IMMUNOASSAY-VERFAHREN UND TEST ZUR ERKENNUNG DER PROLIFERATIVEN DIABETISCHEN RETINOPATHIE
DISPOSITIF MÉDICAL, MÉTHODE D'IMMUNOESSAY ET TEST DE DÉTECTION DE LA RÉTINOPATHIE DIABÉTIQUE PROLIFÉRATIVE

(30) Priority: 22.03.2017 IT 201700031721
(43) Date of publication of application: 26.09.2018
(73) Proprietor: Medical Strips Srl, 00144 Roma (IT)
(72) Inventor: COSTAGLIOLA, Ciro, 86100 Campobasso (IT); GELSO, Aldo, 80122 Napoli (IT); PORCELLINI, Antonio, 80131 Napoli (IT); TECCE, Mario Felice, 80136 Napoli (IT); DELL'OMO, Roberto, 86100 Campobasso (IT)
(74) Representative: Sangiacomo, Ines

(56) References cited:
- US-A1- 2011 269 248
- WANG JHIH-CHENG ET AL: "Detection of low-abundance biomarker lipocalin 1 for diabetic retinopathy using optoelectrokinetic bead-based immunosensing", BIOSENSORS AND BIOELECTRONICS, ELSEVIER BV, NL, vol. 89, 10 November 2016 (2016-11-10), pages 701-709, XP029844943, ISSN: 0956-5663, DOI: 10.1016/J.BIOS.2016.11.014
- CSOSZ ÉVA ET AL: "Diabetic retinopathy: Proteomic approaches to help the differential diagnosis and to understand the underlying molecular mechanisms", JOURNAL OF PROTEOMICS, vol. 150, 1 July 2016 (2016-07-01), pages 351-358, XP029814809, ISSN: 1874-3919, DOI: 10.1016/J.JPROT.2016.06.034

## Description

### Field of the Invention

In its most general aspect the present invention relates to the medical field and in particular the invention relates to a medical device, an immunoassay method and test for detecting (diagnosing) proliferative diabetic retinopathy in a patient.

### State of the Art

Diabetes mellitus, often referred to with the term diabetes alone, is a chronic disease characterized by the presence of high blood glucose levels (hyperglycemia) and due to an altered amount or function of insulin.

Insulin is a hormone produced by the pancreas, allowing the glucose entering the cells and its consequent use as energy source. When this mechanism is altered, the glucose accumulates in the blood circulation.

The diabetes mellitus is distinguished in type 1 diabetes and type 2 diabetes.

The type 1 diabetes is caused by the failure to produce endogenous insulin and affects about 10% of patients. The type 2 diabetes, instead, is caused by an insufficient production of insulin and/or by the inability of the target cells to respond to insulin and affects about 90% of diabetic patients, thus constituting the most widespread form of the disease.

The causes of the diabetes mellitus have not yet been completely identified and defined, however it was found that type 2 diabetes in general develops after 40 years of age, even if cases have been observed in significantly younger patients.

It was also found that some factors favor the development of type 2 diabetes, such as the familiarity (40% of the diabetic patients has a first-degree relative with the same disease), lifestyle (obesity and sedentary life) and the increase in average life.

Furthermore it was found that the prevalence of type 2 diabetes is age-specific: lower than 10% up to 60 years old, ranging from 10% to 20% between 60 and 69 years old, ranging from 15% to 20% beyond 70 years old.

Based on these data, in Europe the risk to develop the diabetes mellitus is around 30-40% *(*DECODE Study Group, Diabetes Care, 2003, 26, 61*).*

Currently the patients suffering from diabetes mellitus are about 371 million worldwide, and this value is supposed to increase according to forecasts estimating 500 million patients that will be affected by diabetes mellitus in 2030 *(*The diabetes pandemic. Lancet. July 9th 2011; 378: 99*)*, a real pandemic.

According to the latest data available, in Italy the people suffering from diabetes mellitus exceeded 3 million, of which 90% is suffering from type 2 diabetes, with a prevalence of 5.5% and an increase of 45% with respect to the 2000s data.

To the above it has to be added that the number of people affected by non-diagnosed diabetes mellitus is estimated to be about 1 million and the number of people suffering from pre-diabetes mellitus, i.e. suffering from glucose intolerance or impaired glycaemia without food *(*Consoli A., Italian Barometer Diabetes Report 2013, Italian Barometer Diabetes Observatory Foundation*)* is estimated to be about 2.6 million.

The prevalence of the diabetes mellitus increases with the age and in the people being 75 years old or older, furthermore, the prevalence of the diabetes mellitus in Italy reached the value of 20.3%, analogously with what occurs worldwide *(Istat, 2012).*

By considering the geographical distribution in Italy, the prevalence of the diabetes mellitus increases going from North to South: 4.9% Northern Italy - 5.5% Center of Italy - 6.2% Southern Italy and Islands.

It has also been observed how the female sex and belonging to lower social classes (indicator of obesity and reduced physical activity) increase the risk of diabetes mellitus.

The chronic-degenerative complications of the diabetes mellitus are predominantly vascular and neurological: macroangiopathy, microangiopathy and neuropathy. Among them the microangiopathy is certainly the most subtle, supporting all those anatomical and functional alterations which are the basis of the organ damage and which are then clinically concretized in a large number of rather important morbid conditions, as acute myocardial infarction, chronic ischemic heart disease, heart failure, brain stroke, chronic renal failure, retinopathy, vascular dementias etc.

Diabetic retinopathy is a severe complication of the diabetes mellitus affecting the retina and, in working age, is the first cause of hypovision and blindness in the industrialized Countries.

It is estimated that a retinopathy has been diagnosed to about one third of the diabetics. The symptoms related thereto often appear late, when the lesions are already advanced, and this often limits the effectiveness of the related treatments.

The diabetic retinopathy is an important cause of blindness and occurs as a result of a damage accumulated in the long run charged to the small blood vessels of the retina. The diabetic retinopathy has been responsible for about 2% of the visual impairment (moderate or severe) and 2.6% of blindness in 2010.

It is estimated that the prevalence of each type of retinopathy in people suffering from diabetes is 35%, whereas that of the proliferative form of the diabetic retinopathy (the most dangerous for the sight) is equal to about 7% *(*Wong TY et al, MESA Study, AJO, 2006*).*

The main risk factors associated to an earlier and faster appearance of the diabetic retinopathy are the duration of the diabetes mellitus, the glycemic failure and the possible concomitant arterial hypertension, both in patients suffering from type 1 diabetes and those suffering from type 2 diabetes.

The blindness caused by diabetic retinopathy could be avoided in more than half the cases if correct patient information and adequate forms of health education were implemented, which are fundamental to the success of any policy of prevention of the visual damage in patients affected by diabetes mellitus.

The screening of the eye complications, with techniques having proven effectiveness and used by trained personnel, allows early detecting the high-risk diabetic retinopathy and thus preventing the loss of sight. However the costs to make a reliable screening are high.

Recently, data on the costs above have been published, according to which the cost per each analyzed individual is about 100 $ (US dollars), whereas each highlighted ocular anomaly affects for about 200 $, and detecting a patient suffering from diabetic retinopathy involves a cost of about 500 $ *(Byrne MM, Parker DF, Tannenbaum SL, Ocasio MA, Lam BL, Zimmer-Galler I, Lee DJ. Cost of a community-based diabetic retinopathy screening program. Diabetes Care. 2014; 37: e236-7).*

These are hardly sustainable costs, also because the screening should be repeated every 2 years, on the population of both sexes from 40 years old *(*Lund SH, Aspelund T, Kirby P, Russell G, Einarsson S, Palsson O, Stefansson E. Individualised risk assessment for diabetic retinopathy and optimisation of screening intervals: a scientific approach to reducing healthcare costs. Br J Ophthalmol. 2016; 100: 683-7*).*

Purpose of the screening is to anticipate the diagnosis of diabetic retinopathy so as to improve its prognosis. However it is a practice whose effectiveness is strongly influenced by the percentage of subscriptions, in its turn also determined by the number of the studied population.

The wider the population the more difficult it will be to reach high percentages of subscriptions. Great resources are used to examine many healthy people with the aim of finding the few which are ill. For this reason, even the smallest reduction of costs has significant impact.

An aid to this problem has come from the recent methodologies of telemedicine applied to ophthalmology. The telemedicine consists in sending photos of the eyes of the patients to a reading center manned by personnel trained to recognize the signs of diabetic retinopathy, so as to detect the subjects to be subjected to an in-depth evaluation and to the possible initiation of the relative treatments.

The automated screening methodologies above for the diabetic retinopathy, despite representing an immense opportunity to manage the increasing demand for retinal diagnostics, are not yet fully validated by the scientific world *(*Sim DA, Mitry D, Alexander P, Mapani A, Goverdhan S, Aslam T, Tufail A, Egan CA, Keane PA. The Evolution of Teleophthalmology Programs in the United Kingdom: Beyond Diabetic Retinopathy Screening. J Diabetes Sci Technol. 2016; 10: 308-17*).*

Furthermore, the problems of the cost of the camera and for the acquisition of the images, reading by specialized personnel, and last but not least, the safety and privacy for the data transmission, remain.

Therefore, at present, in the known art the need to have a quick, cheap and repeatable solution for an early diagnosis of the proliferative diabetic retinopathy is still essentially dissatisfied.

Document US 2011/269248 discloses bio-markers for diagnosing diabetic retinopathy, a use of proteins, whose expression level down-regulated or up-regulated in the tears of a non-proliferative diabetic retinopathy (NPDR) patient, as bio-markers for diagnosing diabetic retinopathy, and a composition and kit for diagnosing diabetic retinopathy comprising antibodies against said proteins.

WANG JHIH-CHENG ET AL: "Detection of low-abundance biomarker lipocalin 1 for diabetic retinopathy using optoelectrokinetic bead-based immunosensing", BIOSENSORS AND BIOELECTRONICS, ELSEVIER BV, NL, vol. 89, 10 November 2016 (2016-11-10) pages 701-709, XP029844943, discloses an optoelectrokinetic bead based immunosensing technique to detect the diabetic retinopathy biomarker LCN1.

CSOSZ EVA ET AL: "Diabetic retinopathy: Proteomic approaches to help the differential diagnosis and to understand the underlying molecular mechanism", JOURNAL OF PROTEOMICS, vol. 150, pages 351-358, XP029814809, discloses the administration of advanced proteomics techniques used in tear biomarker studies and the identified biomarkers with potential to improve the already existing screening methods for diabetic retinopathy detection.

### Summary of the Invention

The technical problem underlying the present invention is to provide means for detecting proliferative diabetic retinopathy in a patient, or however means for a screening of the proliferative diabetic retinopathy, able to overcome one or more of the drawbacks mentioned above with reference to the known art.

Within the scope of some embodiments of the present invention, the aforesaid problem is solved by a method for detecting proliferative diabetic retinopathy in a patient, comprising the steps of:
providing a biological sample of a patient, wherein said sample is taken from lacrimal fluid of said patient;
verifying if predetermined threshold values of three predetermined proteins corresponding to: TNF-alpha, Lipocalin-1 and Lactoferrin, are exceeded in the aforesaid sample, wherein exceeding at least two out of three of the aforesaid threshold values is indicative of the presence, or the likely presence, of proliferative diabetic retinopathy in the aforesaid patient, in which the step of verifying in the aforesaid sample is not carried out on the body of the aforesaid patient, i.e. it is not carried out on the human body.

The aforesaid threshold values in the lacrimal fluid of the patient have been detected to be the following preferred values: 3 pg/ml for TNF-alpha, 1.6 mg/ml for Lipocalin-1 and 2.7 mg/ml for Lactoferrin.

In fact, the authors of the present invention have found, in the lacrimal fluid of a sample of patients affected by diabetes mellitus, that the concentrations of the TNF-alpha, Lactoferrin and Lipocalin-1 proteins are higher in a population of the aforesaid sample affected by proliferative diabetic retinopathy, and have further determined threshold values of the concentration for each of the aforesaid proteins, above which, or better above at least some of them, it can be considered that a given patient is affected by proliferative diabetic retinopathy, with a sensitivity of the method equal to 85% and a specificity of the same equal to 70% with respect to the population not affected by proliferative retinopathy.

In particular, for a given patient, exceeding at least two of the aforesaid threshold values, independently one to another, is a sign of the likely presence in the aforesaid given patient of the proliferative diabetic retinopathy, whereas exceeding all three of the aforesaid threshold values is a sign that the given patient is almost certainly, if not certainly, affected by proliferative diabetic retinopathy.

Within the scope of other embodiments of the present invention, the aforesaid problem is solved by a medical device comprising three pathways, wherein each pathway is extending between a first zone intended to receive a sample in the liquid form and an end zone having an absorbent portion, wherein each pathway comprises the following zones, all extending between the aforesaid first zone and the aforesaid end zone:
an intermediate zone, or catch zone, loaded with a predetermined quantity of a predetermined protein, the aforesaid protein being constituted by TNF-alpha, Lactoferrin and Lipocalin-1 in the aforesaid pathways, respectively;
a zone arranged upstream of the aforesaid intermediate zone, or conjugate zone, loaded with a respective antibody bounded to an indicator means, wherein the aforesaid antibody is specific for the TNF-alpha, Lactoferrin and Lipocalin-1 protein in the aforesaid pathways, respectively;
a zone arranged downstream of the aforesaid intermediate zone, or control zone, loaded with an anti-immunoglobulin species-specific antibody.

Preferably, the aforesaid device comprises a support which the aforesaid three pathways are combined with and preferably constrained with.

Preferably, the aforesaid three pathways are capillary pathways.

Preferably, the aforesaid three pathways are lateral-flow pathways.

Preferably, the aforesaid three pathways comprise a nitrocellulose layer.

Preferably, the aforesaid indicator means comprises colored microbeads.

Preferably, the aforesaid respective protein is loaded in solid phase in the aforesaid catch zone.

Preferably, the aforesaid respective antibody is loaded in solid phase in the aforesaid control zone.

Preferably, in the aforesaid catch zone of each of the aforesaid pathways, the aforesaid protein is respectively present in amounts higher than the expected ones, thus exceeding the aforesaid threshold values.

Within the scope of other embodiments of the present invention, the problem above is solved by an immunoassay test of the TNF-alpha, Lipocalin-1 and Lactoferrin proteins for detecting the proliferative diabetic retinopathy in a patient, comprising:
providing a sample lacrimal fluid from a patient;
loading the aforesaid sample on a medical device of the aforesaid type;
verifying if predetermined threshold values of three predetermined proteins corresponding to: TNF-alpha, Lipocalin-1 and Lactoferrin, are exceeded in the aforesaid sample, wherein exceeding at least two out of three of the aforesaid threshold values is indicative of the presence, or the likely presence, of proliferative diabetic retinopathy in the aforesaid patient, in which the step of verifying in the aforesaid sample is not carried out on the body of the aforesaid patient, i.e. is not carried out on the human body.

The aforesaid threshold values, particularly in the lacrimal fluid of the aforesaid sample, have been detected to be the following preferred values: 3 pg/ml for TNF-alpha, 1.6 mg/ml for Lipocalin-1 and 2.7 mg/ml for Lactoferrin.

Preferably, the aforesaid sample loaded on the aforesaid device is taken from saliva, serum, plasma, urine, lacrimal fluid of the aforesaid patient.

In practice, the present invention provides means allowing a large scale screening for detecting in an early step, quickly, repeatably and with low costs, the proliferative diabetic retinopathy.

Within the scope of the present invention the expressions patient, subject and individual are interchangeable and denote a living being, for example a human, from which the aforesaid biological sample comes from.

### Brief description of the figures

Further characteristics and advantages of the invention will be more evident by reviewing the following detailed description of some preferred, but not exclusive, embodiments, depicted for illustration purposes only and without limitation, with the aid of the accompanying drawings, wherein:
- figure 1 schematically depicts a view from above of an embodiment of a medical device for diagnosing the proliferative diabetic retinopathy in a patient, in accordance with the present invention;
- figure 2 schematically depicts a detail with an enlarged scale of the device of figure 1;
- figure 3, in the images a-f, shows the logistic analysis, the ROC curve and the comparison of the found values of an ELISA immunoassay test on multi-well plates for the TNF-alpha protein;
- figure 4, in the images a-f, shows the logistic analysis, the ROC curve and the comparison of the found values of an ELISA immunoassay test on multi-well plates for the Lipocalin-1 protein;
- figure 5, in the images a-f, shows the logistic analysis, the ROC curve and the comparison of the found values of an ELISA immunoassay test on multi-well plates for the Lactoferrin protein;
- figure 6, in the images a and b, shows specificity and sensitivity of an immunoassay test for detecting proliferative diabetic retinopathy, the test being carried out by a medical device in accordance with the present invention, by means of contingency tables through the comparison between healthy subjects (control), diabetic subjects not suffering from proliferative retinopathy and diabetic subjects suffering from proliferative retinopathy, wherein the medical device showed diagnostic power equal to that of ELISA test.

### Detailed description of the invention

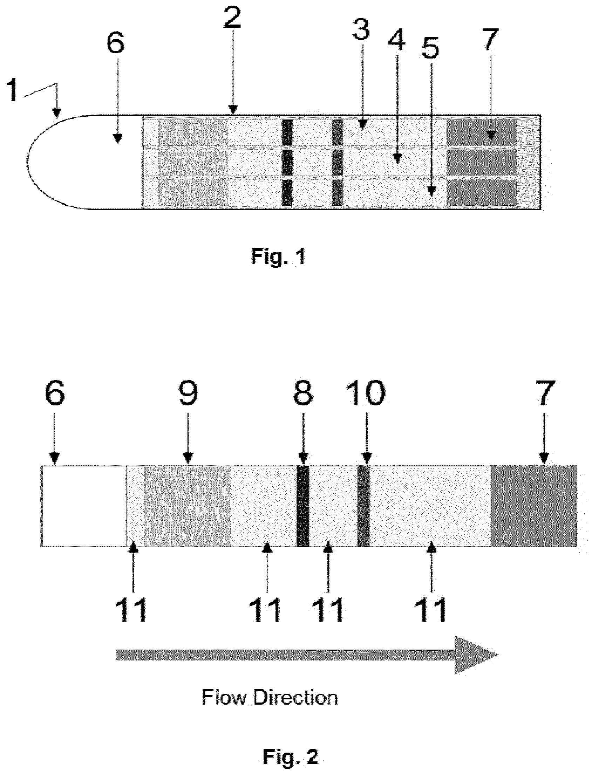

With reference to figure 1 a medical device in accordance with the present invention is denoted as a whole by **1.**

The device **1** comprises a support **2** and three pathways **3, 4** and **5** combined with, and extending on, the support **2.**

The three pathways **3, 4** and **5** are essentially similar to each other and therefore the related description is made with reference to one pathway only, as depicted in the example of figure 2.

In particular, the example of figure 2 depicts a pathway extending between a first zone **6** intended to receive a sample in the liquid form, and an end zone **7** wherein, for receiving the sample in the liquid form, the first zone **6** comprises a porous pad, whereas, for completing the pathway, the end zone **7** is equipped with an absorbent portion.

Between the first zone **6** and the end zone **7,** the pathway depicted in the example of figure 2, and therefore each of the pathways **3, 4** and **5,** comprises an intermediate zone also identified as a catch zone and denoted by **8,** which is loaded with a predetermined protein.

In detail, in the pathways **3, 4** and **5** the catch zone **8** is loaded respectively with TNF-alpha, Lactoferrin and Lipocalin-1.

The amount of the proteins is: TNF-alpha not less than 1 pg, Lactoferrin not less than 200 µg and Lipocalin-1 not less than 100 µg.

Between the first zone **6** and the catch zone **8,** i.e. upstream of the latter, a conjugate zone **9** is included, which is loaded with a respective antibody bounded to an indicator means.

In detail, in the pathways **3, 4** and **5,** the conjugate zone 9 is loaded with an antibody specific for the TNF-alpha protein, Lactoferrin protein and Lipocalin-1 protein, respectively.

Between the catch zone **8** and the end zone **7,** i.e. downstream of the catch zone **8,** a control zone **10** is included, which is loaded with an anti-immunoglobulin species-specific antibody.

Furthermore, it has to be said that preferably both the aforesaid proteins and the aforesaid antibodies are loaded in solid phase in the respective zones.

As depicted in the examples of the figures, in each pathway **3, 4** and **5** separation zones are further provided and depicted by **11,** which are arranged between the first zone **6** and the conjugate zone **9,** between the conjugate zone **9** and the catch zone **8,** between the catch zone **8** and the control zone **10** and between the control zone **10** and the end zone **7.**

Furthermore, with reference to the three pathways **3, 4** and **5** it has to be added that preferably they are capillary pathways, and even more preferably they are lateral-flow pathways.

Particularly, the pathways **3, 4** and **5** comprise, or are constituted by, one nitrocellulose layer advantageous in making the capillary, lateral-flow pathways.

For what concerns the aforesaid indicator means, it has to be said that it preferably comprises colored microbeads, for example of the type of Estapor® Microspheres marketed by Merck Millipore, which are able - by emitting color - to highlight the catch zone and/or the control zone. If the catch zone is not colored and the control zone is colored, it means that the analyte concentration is higher than that pre-set by the test (positive test to the proliferative retinopathy); vice versa, if both the catch and control zones are colored, the test is negative.

In practice, the embodiment depicted in the examples of figures 1 and 2 shows a medical device allowing an early diagnosis, or however a pre-diagnosis, of the proliferative diabetic retinopathy by a diagnostic procedure having high sensitivity and specificity, simple to be carried out and having low cost, which can be used for carrying out large scale screening consisting in the rapid and simultaneous immunoassay in the lacrimal fluid or in other biological fluids of the aforesaid three proteins, whose concentration is related to the progress of the proliferative diabetic retinopathy, whereby on the basis of the exceeding of threshold values suitably identified, it is possible to selectively identify, in a group of individuals, with instantaneous reading as part of a clinical examination, those to subject to further ophthalmologic investigations.

Thus, in accordance with what above and according to the present invention, an immunoassay test of the TNF-alpha, Lipocalin-1 and Lactoferrin proteins for detecting proliferative diabetic retinopathy in a patient has been also provided, comprising:
providing a sample of a biological liquid of a patient;
loading the sample on a medical device of the aforesaid type described with reference to the examples of figures 1 and 2;
verifying if predetermined threshold values of three predetermined proteins corresponding to: TNF-alpha, Lipocalin-1 and Lactoferrin, are exceeded in the sample, wherein exceeding at least two out of three of the aforesaid threshold values is indicative of the presence, or the likely presence, of proliferative diabetic retinopathy in the patient, in which the step of verifying in the sample is not carried out on the body of the patient, i.e. it is not carried out on the human body.

The three preferred threshold values, in particular in the lacrimal fluid of the patient, have been detected being: 3 pg/ml for TNF-alpha, 1.6 mg/ml for Lipocalin-1 and 2.7 mg/ml for Lactoferrin.

Anyway the liquid sample can be constituted by or also taken, for example, from saliva, serum, plasma, or urine of the patient.

It is understood that in accordance with the present invention, devices that are different to that depicted in the examples of figures 1 and 2 can be used, given that the present invention provides a correlation among the concentrations of the TNF-alpha, Lactoferrin and Lipocalin-1 proteins for detecting subjects (patients) suffering from proliferative diabetic retinopathy, or however at risk of proliferative diabetic retinopathy.

Therefore, in accordance with the present invention, a method for detecting proliferative diabetic retinopathy in a patient is also provided, comprising the steps of:
providing a biological sample of a patient, wherein said sample is taken from lacrimal fluid of said patient;
verifying if predetermined threshold values of three predetermined proteins corresponding to TNF-alpha, Lipocalin-1 and Lactoferrin have been exceeded in the aforesaid sample, wherein exceeding at least two out of three of the aforesaid threshold values is indicative of the presence, or the likely presence, of proliferative diabetic retinopathy in the aforesaid patient, in which the step of verifying in the sample is not carried out on the body of the patient, i.e. it is not carried out on the human body.

The aforesaid threshold values, particularly in the lacrimal fluid of the patient, have been detected being: 3 pg/ml for TNF-alpha; 1.6 mg/ml for Lipocalin-1 and 2.7 mg/ml for Lactoferrin.

To determine the threshold values we proceeded as follows.

### Example 1: Preparation of an ELISA immunoassay test on multi-well plates for each of the TNF-alpha, Lipocalin-1 and Lactoferrin proteins

### - Preparation of the immunoassay, tests, results.

90 diabetic patients and 44 healthy subjects have been used. The classification of diabetic retinopathy has been carried out according to ETDRS criteria *("*Grading diabetic retinopathy from stereoscopic color fundusphotographs-an extension of the modified Airlie House classification. ETDRS report number 10. Early treatment diabetic retinopathy study research group," Ophthalmology, vol. 98, supplement 5, pp. 786-806, 1991). The diabetic patients have been divided in 2 groups: 45 patients showed non-proliferative retinopathy, whereas 45 patients were diagnosed as being affected by proliferative diabetic retinopathy.

A lacrimal secretion has been collected from each eye of each patient by using a standard stripe of the Schirmer test. The stripe of bibulous graph paper has been positioned in the lower conjunctival fornix, at the outer corner, for 5 minutes, waiting that it was dampened by capillarity. After the withdrawal, the stripe has been immediately transferred to a sterile tube labeled and frozen at -40°C until the analysis. The wet part of the stripe of each Schirmer test was then cut in small pieces and soaked in 50 *µ*L/5mm saline buffer (PBS) for 3 h in order to elute the lacrimal proteins.

The TNF-alpha protein has been determined by using a 96-well plate coated with antibody specific for human TNF-alpha (sensitivity 0.61 pg/ml), according to manufacturer instructions (human TNF-alpha ELISA Kit SEA133Hu, USCN, distributed by: DBA, Segrate-Milano, Italy).

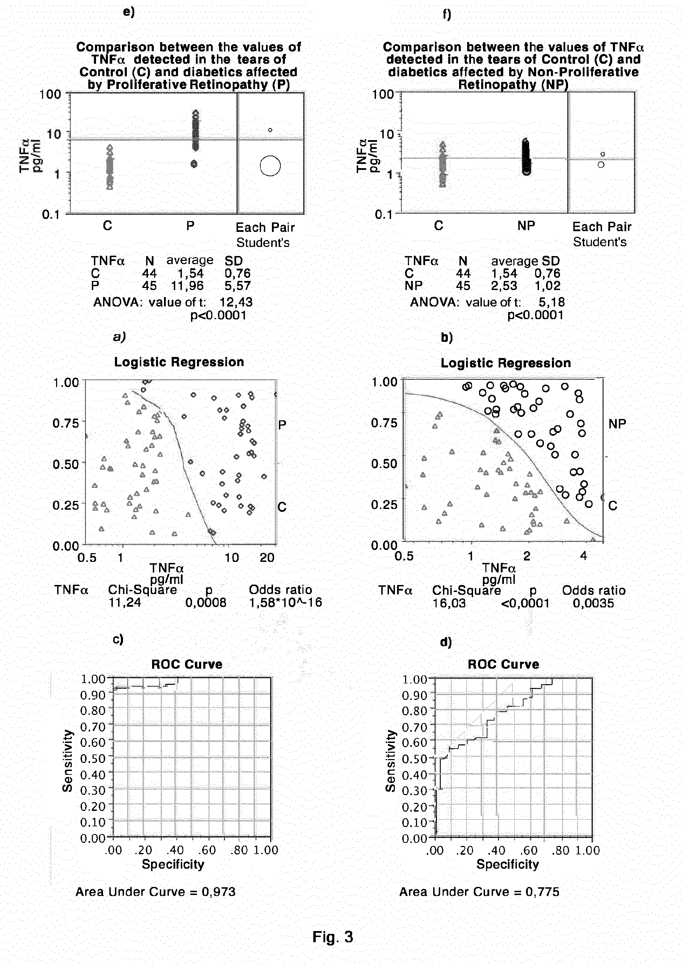

Figure 3 shows the logistic analysis, the ROC curve and the comparison between the averages of the values found in healthy subjects (C), diabetics affected by non-proliferative retinopathy (NP) and diabetics affected by proliferative retinopathy (P).

It has been considered normal a value of TNF-alpha up to 3 pg/ml.

The Lipocalin-1 protein has been determined by using a 96-well plate coated with antibody specific for human LCN1 (Lipocalin-1)(sensitivity 0.39 ng/ml), according to manufacturer instructions (ELISA Kit CSB-EL012810, Cusabio, distributed by: Space Import Export srl, Milano, Italy).

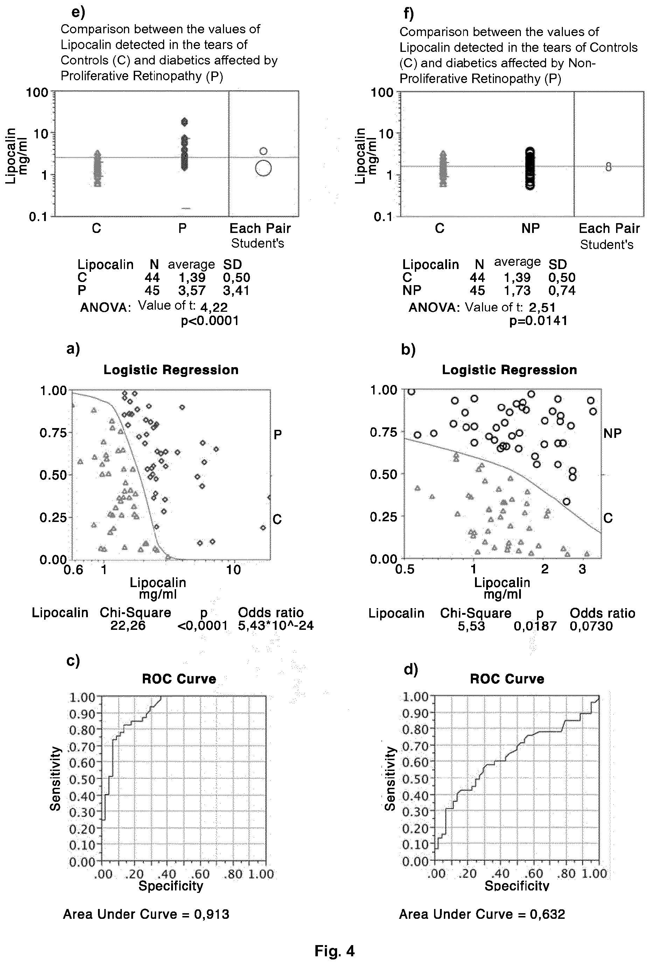

Figure 4 shows the logistic analysis, the ROC curve and the comparison between the averages of the values found in healthy subjects (C), diabetics affected by non-proliferative retinopathy (NP) and diabetics affected by proliferative retinopathy (P).

It has been considered normal a value of Lipocalin-1 up to 1.6 mg/ml.

The Lactoferrin protein has been determined by using a 96-well plate coated with antibody specific for human TRFL (Lactoferrin)(sensitivity 0.78 ng/ml), according to manufacturer instructions (ELISA Kit CSB-E08831H, Cusabio, distributed by: Space Import Export srl, Milano, Italy).

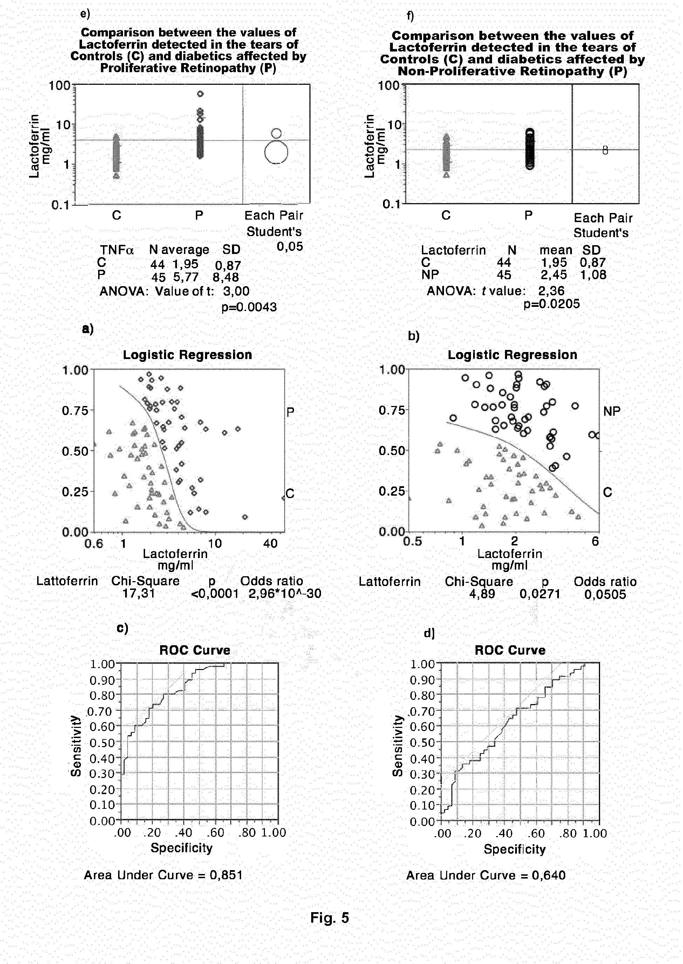

Figure 5 shows the logistic analysis, the ROC curve and the comparison between the averages of the values found in healthy subjects (C), diabetics affected by non-proliferative retinopathy (NP) and diabetics affected by proliferative retinopathy (P).

It has been considered normal a value of Lactoferrin up to 2.7 mg/ml.

### Example 2: Preparation of nitrocellulose stripes for the simultaneous dosing of the three TNF-alpha, Lipocalin-1 and Lactoferrin proteins

### - Preparation of the stripes, tests, results.

Three tests on nitrocellulose stripe (Millipore) having lateral flow for detecting the three TNF-alpha, Lipocalin-1 and Lactoferrin proteins present in the lacrimal fluid at concentrations above the identified minimum limit, have been fine-tuned. The methodology has been prepared in the competitive mode according to the scheme below that provides the use of a stripe (pathway) for immunological assays ("immunostrip"), individually prepared for each of the three proteins as previously depicted with reference to the example of figure 2 to which description reference is made.

Three different stripes can then be allocated together on a unique support for being jointly used for dosing and subsequent overall reading the results, as previously depicted with reference to the example of figure 1 to which description reference is made.

The samples of lacrimal fluid are placed on a porous pad (first zone) from which a capillary lateral flow starts through the nitrocellulose, being generated by the same liquid of the sample, in the direction of the other end of the stripe where there is an absorbing pad (end zone). Thereby, the components of the sample are transported to the next zone of the stripe, wherein conjugates constituted by colored microbeads bonded to antibodies specific for the protein under testing (conjugate zone) have been deposited. These conjugates enter the solution as a flow contacting the components thereof. Thus, if there is the protein under testing in the sample, it will be able to complex with the colored conjugates and the lateral flow will transport this material to a next zone where there is a catch line (catch zone). In the catch line there is, in solid phase and irreversibly fixed to the nitrocellulose, a defined amount of the protein under testing. Thus, if in the sample there is such an amount of the protein to saturate the antibody related thereto, conjugated to the colored microbeads, this will not be able to bond the same protein in solid phase and will further proceed with the lateral flow. In the absence of the protein derived from the sample, the conjugate bonds to that protein in solid phase instead, by coloring the catch line. Upon reaching a next area wherein there is a control stripe (control zone), an anti-immunoglobulin species-specific antibody (in the particular case, rabbit anti-immunoglobulins) being adhered here in solid phase, the residual microbeads-antibody complex, if part has already been bonded by the total catch line, if the sample is positive, i.e. if the concentration of the analytes is equal to or higher than the predetermined one, however will be bonded to the control line that will be colored in turn.

In practice, if in the lacrimal fluid there is a concentration of the protein under testing higher than the defined value, the catch line will not be colored by the microbeads conjugated to specific antibodies, but only the next control line will be colored where the conjugate will be bonded anyway (positive result, coloring only of the control line). If instead the protein is absent or it has a lower concentration than the defined value, the conjugate will be able to color the catch line and also the next control line (negative result, coloring both of the catch line and the control line).

The colored microbeads have been used according to manufacturer specifications (Merck Millipore, Estapor® Microspheres). The primary antibodies used are polyclonal ones derived from rabbits (Sigma-Aldrich). However the same can be obtained with a cycle of antigenic administrations in rabbit, mouse or goat of each of the three purified proteins or fragments thereof. The quality of the antibodies can possibly be further increased for immunoaffinity. The secondary antibody (anti-rabbit immunoglobulin) has been used according to manufacturer specifications (Sigma-Aldrich). The fine-tuning of the preparation of the stripes has essentially concerned comparative tests with the immunoassays carried out on multi-well plate in order to determinate the amounts of reagents, in particular the conjugates, to be added on the stripes so as to be able to obtain positivity only when the established cut-off values are exceeded, i.e. of at least two out of three threshold values.

The data of the tests carried out with the stripes having lateral flow were widely overlapping those deriving from the immunoassays on multi-plate. In fact the stripes configured to detect the three proteins, at concentrations above the threshold values previously determined, detected the same results in almost all (97.5%) the cases analyzed, with the advantage that they do not require devices and laboratory equipment and they require minimal manual execution for carrying out the tests.

In particular, in the 90 diabetic patients what follows has been detected, with values above the cut-off:
a) for Lactoferrin: 53 by strip-test and 57 by ELISA;
b) for Lipocalin-1: 63 by strip-test and 73 by ELISA;
c) for TNF-alpha: 57 by strip-test and 59 by ELISA.

By comparing overall the multi-well methodology with the rapid test:
ELISA: out of the 45 proliferative patients, 29 with triple positivity; 11 with double positivity, making a total of 40/45 identified patients (88.8%) and 22/90 false positives.
Stripe having lateral flow: out of the 45 proliferative patients, 29 with triple positivity; 10 with double positivity, making a total of 39/45 identified patients (86.6%) and 23/90 false positives.

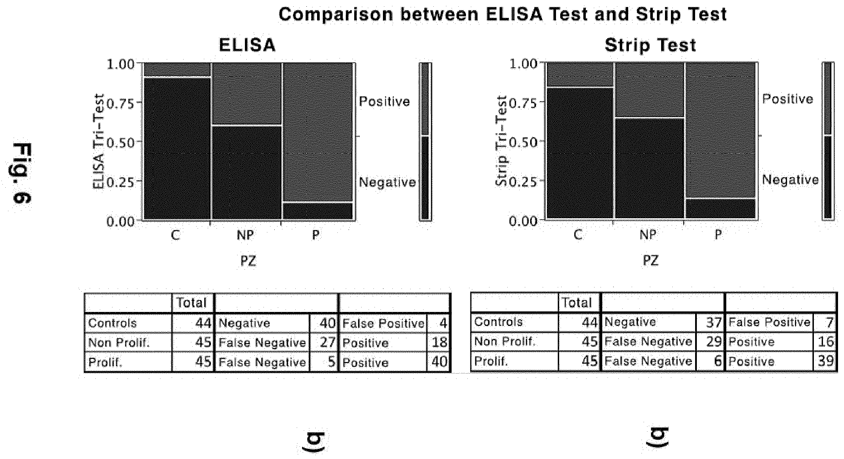

Thus, by and large with the flow stripes (Strip Tri-Test) it is possible to detect 97.5% diagnostic positivity compared to what has been seen with ELISA, as visible in figure 6.

To the present invention, in the illustrated and described embodiments, in order to satisfy contingent and specific requirements, a person skilled of the art can make a number of variations and changes, on the other hand all contained in the scope of protection of the invention as defined by the following claims.

## Claims

1. Medical device (1) comprising three pathways (3, 4, 5), wherein each pathway (3, 4, 5) of said three pathways is extending between a first zone (6) intended to receive a sample in the liquid form and an end zone (7) having an absorbent portion, wherein each of said pathways (3, 4, 5) comprises the following zones, all extending between said first zone (6) and said end zone (7):
an intermediate zone or catch zone (8) loaded with a predetermined quantity of a predetermined protein, said protein being constituted by TNF-alpha, Lactoferrin and Lipocalin-1 in said pathways (3, 4, 5), respectively;
a conjugate zone (9) arranged upstream of said intermediate zone (8) and loaded with a respective antibody bounded to an indicator means, wherein said antibody is specific for the TNF-alpha, Lactoferrin and Lipocalin-1 proteins in said pathways (3, 4, 5), respectively;
a control zone (10) arranged downstream of said intermediate zone and loaded with an anti-immunoglobulin species-specific antibody.

2. Device according to claim 1, comprising a support (2) which said three pathways (3, 4, 5) are combined with.

3. Device according to claim 1 or 2, wherein said three pathways (3, 4, 5) are capillary pathways.

4. Device according to any one of the preceding claims, wherein said three pathways (3, 4, 5) are lateral-flow pathways.

5. Device according to any one of the preceding claims, wherein said three pathways (3, 4, 5) comprise a nitrocellulose layer.

6. Device according to any one of the preceding claims, wherein said indicator means comprises colored microbeads.

7. Device according to any one of the preceding claims, wherein said protein is loaded in solid phase in said catch zone (8).

8. Device according to any one of the preceding claims, wherein said antibody is loaded in solid phase in said control zone (10).

9. Method for detecting proliferative diabetic retinopathy in a patient, comprising the steps of:
providing a biological sample of a patient, wherein said sample is taken from lacrimal fluid of said patient;
verifying if predetermined threshold values of three predetermined proteins corresponding to: TNF-alpha, Lipocalin-1 and Lactoferrin, are exceeded in said sample, wherein exceeding at least two out of three of said threshold values is indicative of the presence, or the likely presence, of proliferative diabetic retinopathy in said patient, the step of verifying in said sample not being carried out on the body of said patient.

10. Method according to claim 9, wherein said threshold values correspond to: 3 pg/ml for TNF-alpha, 1.6 mg/ml for Lipocalin-1 and 2.7 mg/ml for Lactoferrin.

11. Immunoassay test of the TNF-alpha, Lipocalin-1 and Lactoferrin proteins for detecting proliferative diabetic retinopathy in a patient, comprising:
providing a sample of lacrimal fluid from a patient;
loading said sample on a medical device according to any one of claims 1-8;
verifying if predetermined threshold values of three predetermined proteins corresponding to: TNF-alpha, Lipocalin-1 and Lactoferrin, are exceeded in said sample, wherein exceeding at least two out of three of said threshold values is indicative of the presence, or the likely presence, of proliferative diabetic retinopathy in said patient, the step of verifying in said sample not being carried out on the body of said patient.

12. Test according to claim 11, wherein said threshold values correspond to: 3 pg/ml for TNF-alpha, 1.6 mg/ml for Lipocalin-1 and 2.7 mg/ml for Lactoferrin.

## Patentansprüche

1. Medizinische Vorrichtung (1) mit drei Wegstrecken (3, 4, 5), wobei jede Wegstrecke (3, 4, 5) der drei Wegstrecken sich zwischen einer ersten Zone (6), die dazu bestimmt ist, eine Probe in flüssiger Form aufzunehmen, und einer Endzone (7) mit einem absorbierenden Abschnitt erstreckt, wobei jeder der Wegstrecken (3, 4, 5) die folgenden Zonen umfasst, die sich alle zwischen der ersten Zone (6) und der Endzone (7) erstrecken:
eine Zwischenzone oder Fangzone (8), die mit einer vorbestimmten Menge eines vorbestimmten Proteins beladen ist, wobei das Protein aus TNF-alpha, Lactoferrin und Lipocalin-1 in den Wegstrecken (3, 4, 5) gebildet wird;
stromaufwärts dieser Zwischenzone (8) ist eine Konjugatzone (9) angeordnet, die mit jeweils einem Antikörper beladen ist, der mit einem Indikatormittel gebunden ist, wobei der Antikörper spezifisch für die TNF-alpha-, Lactoferrin- und Lipocalin-1-Proteine in den genannten Wegstrecken (3, 4, 5) ist;
eine dieser Zwischenzone nachgeordnete und mit einem anti-Immunglobulin-Spezies-spezifischen Antikörper beladene Kontrollzone (10).

2. Vorrichtung nach Anspruch 1, umfassend einen Träger (2), dem die drei Wegstrecken (3, 4, 5) zugeordnet sind.

3. Vorrichtung nach Anspruch 1 oder 2, wobei die drei Wegstrecken (3, 4, 5) Kapillarwege sind.

4. Vorrichtung nach einem der vorhergehenden Ansprüche, wobei die drei Wegstrecken (3, 4, 5) seitliche Flusswege sind.

5. Vorrichtung nach einem der vorhergehenden Ansprüche, wobei die drei Wegstrecken (3, 4, 5) eine Nitrozelluloseschicht umfassen.

6. Vorrichtung nach einem der vorhergehenden Ansprüche, wobei das Indikatormittel farbige Mikrokügelchen umfasst.

7. Vorrichtung nach einem der vorhergehenden Ansprüche, wobei das Protein in fester Phase in der Fangzone (8) beladen ist.

8. Vorrichtung nach einem der vorhergehenden Ansprüche, wobei der Antikörper in fester Phase in der Kontrollzone (10) geladen ist.

9. Verfahren zur Erkennung von proliferativer diabetischer Retinopathie in einem Patienten, umfassend die Schritte:
Bereitstellen einer biologischen Probe eines Patienten, wobei die Probe aus Tränenflüssigkeit des Patienten genommen wird;
Verifizieren, ob vorbestimmte Schwellenwerte von drei vorbestimmten Proteinen wie TNF-alpha, Lipocalin-1 und Lactoferrin in der Probe überschritten werden, wobei das Überschreiten von mindestens zwei von drei der Schwellenwerte das Vorhandensein oder die wahrscheinliche Anwesenheit von proliferativer diabetischer Retinopathie in dem Patienten anzeigt, wobei der Schritt des Verifizierens in der Probe nicht auf dem Körper des Patienten durchgeführt wird.

10. Verfahren nach Anspruch 9, wobei die Schwellenwerte 3 pg/ml für TNF-α, 1.6 mg/ml für Lipocalin-1 und 2.7 mg/ml für Lactoferrin entsprechen.

11. Immunoassay-Test der TNF-alpha-, Lipocalin-1- und Lactoferrin-Proteine zur Detektion von proliferativer diabetischer Retinopathie in einem Patienten, umfassend:
Bereitstellen einer Probe von Tränenflüssigkeit aus einem Patienten;
Laden der Probe auf eine medizinische Vorrichtung nach einem der Ansprüche 1-8;
Verifizieren, ob vorbestimmte Schwellenwerte von drei vorbestimmten Proteinen wie TNF-alpha, Lipocalin-1 und Lactoferrin in der Probe überschritten werden, wobei das Überschreiten von mindestens zwei von drei der Schwellenwerte das Vorhandensein oder die wahrscheinliche Anwesenheit von proliferativer diabetischer Retinopathie in dem Patienten anzeigt, wobei der Schritt des Verifizierens in der Probe nicht auf dem Körper des Patienten durchgeführt wird.

12. Test nach Anspruch 11, wobei die Schwellenwerte 3 pg/ml für TNF-α, 1.6 mg/ml für Lipocalin-1 und 2.7 mg/ml für Lactoferrin entsprechen.

## Revendications

1. Dispositif médical (1) comprenant trois voies (3, 4, 5), dans lequel chaque voie (3, 4, 5) desdites trois voies s'étend entre une première zone (6) destinée à recevoir un échantillon sous la forme liquide et une zone d'extrémité (7) ayant une portion absorbante, dans lequel chaque voie (3, 4, 5) comprend les zones suivantes, s'étendant toutes entre ladite première zone (6) et ladite zone d'extrémité (7):
une zone intermédiaire ou une zone de capture (8) chargée d'une quantité prédéterminée d'une protéine prédéterminée, ladite protéine étant respectivement constituée de TNF-alpha, de Lactoferrine et de Lipocaline-1 dans lesdites voies (3, 4, 5);
une zone conjuguée (9) disposée en amont de ladite zone intermédiaire (8) et chargée d'un anticorps respectif lié à un moyen indicateur, dans laquelle ledit anticorps est respectivement spécifique des protéines de TNF-alpha, de Lactoferrine et de Lipocaline-1 dans lesdites voies (3, 4, 5);
une zone de contrôle (10) disposée en aval de ladite zone intermédiaire et chargée d'un anticorps d'anti-immunoglobuline spécifique de l'espèce.

2. Dispositif selon la revendication 1, comprenant un support (2) auquel lesdites trois voies (3, 4, 5) sont associées.

3. Dispositif selon la revendication 1 ou 2, dans lequel lesdites trois voies (3, 4, 5) sont des voies capillaires.

4. Dispositif selon l'une quelconque des revendications précédentes, dans lequel lesdites trois voies (3, 4, 5) sont des voies à flux latéral.

5. Dispositif selon l'une quelconque des revendications précédentes, dans lequel lesdites trois voies (3, 4, 5) comprennent un couche de nitrocellulose.

6. Dispositif selon l'une quelconque des revendications précédentes, dans lequel ledit moyen indicateur comprend des microbilles colorées.

7. Dispositif selon l'une quelconque des revendications précédentes, dans lequel ladite protéine est chargée en phase solide dans chaque zone de capture (8).

8. Dispositif selon l'une quelconque des revendications précédentes, dans lequel ledit anticorps est chargé en phase solide dans ladite zone de contrôle (10).

9. Méthode de détection de la rétinopathie diabétique proliférante chez un patient, comprenant les étapes de:
fournir un échantillon biologique d'un patient, dans laquelle ledit échantillon est prélevé du liquide lacrymal dudit patient;
contrôler si les valeurs seuils prédéterminées de trois protéines prédéterminées correspondant au: TNF-alpha, Lipocaline-1 et Lactoferrine, sont dépassées dans ledit échantillon, dans lequel le dépassement d'au moins deux des trois valeurs seuils indique la présence ou la présence probable d'une rétinopathie diabétique proliférante chez ledit patient, l'étape consistant à contrôler ledit échantillon n'étant pas effectuée sur le corps dudit patient.

10. Méthode selon la revendication 9, dans laquelle lesdites valeurs seuils correspondent à: 3 pg/ml pour le TNF-alpha, 1,6 mg/ml pour la Lipocaline-1 et 2,7 mg/ml pour la Lactoferrine.

11. Test de dosage immunologique de protéines TNF-alpha, Lipocaline-1 et Lactoferrine pour la détection d'une rétinopathie diabétique proliférante chez un patient, comprenant:
fournir un échantillon de fluide lacrymal d'un patient;
charger ledit échantillon sur un dispositif médical selon l'une quelconque des revendications 1-8;
contrôler si les valeurs seuils prédéterminées de trois protéines prédéterminées correspondant au: TNF-alpha, Lipocaline-1 et Lactoferrine, sont dépassées dans ledit échantillon, dans lequel le dépassement d'au moins deux des trois valeurs seuils indique la présence ou la présence probable d'une rétinopathie diabétique proliférante chez ledit patient, l'étape consistant à contrôler ledit échantillon n'étant pas effectuée sur le corps dudit patient.

12. Test selon la revendication 11, dans laquelle lesdites valeurs seuils correspondent à: 3 pg/ml pour le TNF-alpha, 1,6 mg/ml pour la Lipocaline-1 et 2,7 mg/ml pour la Lactoferrine.
